# EUROPEAN PATENT APPLICATION

(11) **EP 3 187 147 A1**
(43) Date of publication of application: **05.07.2017**
(21) Application number: 14900672.8
(22) Date of filing: 14.10.2014
(51) Int. Cl.: A61F 2/04, A61F 2/848

(54) **DEGRADABLE CONNECTOR IMPLANTED IN HUMAN BODY**

(30) Priority: 29.08.2014 CN 201410436927
(71) Applicant: Dongguan Dianfu Product Design Co., Ltd., Dongguan, Guangdong 523000 (CN)
(72) Inventor: LI, Yangde, Dongguan Guangdong 523000 (CN)
(74) Representative: Hryszkiewicz, Danuta
(86) International application number: PCT/CN2014/088548
(87) International publication number: WO 2016/029534

(57) **Abstract**

A degradable connector (10) implanted in a human body comprises a cylindrical magnesium alloy connection tube (1) and a magnesium alloy line (2). The outer wall of the connection tube (1) is provided with a plurality of warped thorns (3). The connection tube (1) is inserted into broken or damaged inner vessel cavities of the human body, and the warped thorns (3) are inserted into inner walls of the inner vessel cavities of the human body so that the broken or damaged inner vessel cavities of the human body are connected. The magnesium alloy line (2) surrounds the connection tube (1) and the inner vessel cavities of the human body so that the connection tube (1) is fixedly connected to the inner vessel cavities of the human body. The degradable connector (10) connects damaged or broken intestinal canals, blood vessels, lymphatic vessels or nerves, so that wounds are gradually healed, and the connector is gradually degraded or absorbed by the human body.

## Description

The invention relates to a field of medical apparatus and instruments, and more particularly, to a degradable stent which is implanted in human body.

Common diseases on the lumen including vascular embolization, esophageal carcinoma, colorectal cancer, prostatic hyperplasia, and biliary calculi are treated by open surgery which is difficult to operate, and has problems as many complications, high risk, and high recurrence rate, etc. Therefore, it would be useful to develop a simple, safe and effective method to solve the above problems, and a stent implantation is an effective one.

The intraluminal stent is a common medical instrument, and mostly is tubular in shape. After the stent is implanted in the human body, the stent operates to dilate and support the narrow lumens including vessels, esophagus, biliary tract, intestinal tract, and ureter in the human body, relieve obstructions in the lumens, and keep the lumens unobstructed, thus, the stent implantation which leads to less complications is convenient and reliable.

Existing degradable medical stents are mostly made from magnesium alloy. The magnesium alloy is absorbed by the human body within a short time as the wound heals up, thus the endothelial cell proliferation in the lumen caused by long duration of the stent in the human body is avoided.

Conventional magnesium alloy stents are tubular in shape and have smooth surface so that the stents tend to detach from the lumens, or dislocate, or even collapse as the magnesium alloy is absorbed by the human body over time, especially when the patient is doing intense exercises.

In view of the above-described problems, it is one objective of the invention to provide a degradable stent which is implanted in human body. An outer wall of a magnesium alloy tube of the stent is provided with barbs and a magnesium alloy wire. The stent is inserted in a lumen of the human body, and the barbs are inserted in an inner wall of the lumen. The magnesium alloy wire is wound around the magnesium alloy tube and the lumen, thus the magnesium alloy tube is fixedly connected to the lumen of the human body, preventing the magnesium alloy tube from falling off from the lumen of the human body, dislocating, or event collapsing as the magnesium alloy is absorbed by the human body.

The technical solutions of the invention are summarized as follows.

A degradable stent which is implanted in human body, comprises a cylindrical magnesium alloy tube and a magnesium alloy wire. An outer wall of the magnesium alloy tube is provided with a plurality of barbs. The magnesium alloy tube is inserted in a broken or damaged lumen in human body, and the barbs are inserted in an inner wall of the lumen in the human body, thus the stent is connected to the broken and damaged lumen in the human body. The magnesium alloy wire is wound around the magnesium alloy tube and the lumen, thus the magnesium alloy tube is fixedly connected to the lumen in the human body.

In a class of this embodiment, the magnesium alloy tube comprises a main body and at least one branch. The main body communicates with the branch. Sizes and shapes of the main body and the branch are tailored according to actual conditions of the lumen in the human body, for example, the main body and the branch form T-shaped bifurcation, a Y-shaped bifurcation, or an amorphous bifurcation.

In a class of this embodiment, a joint between the main body and the branch is coated with a magnesium alloy protection layer. Bifurcations of the lumens in the human body (including vessel, lymphatic tube, nerve, or biliary tract) are the positions which bear the most frequent blood rush or body fluid rush, thus the magnesium alloy tube placed at the bifurcations is more likely to break or collapse. Therefore, a magnesium alloy protection layer is applied to the magnesium alloy tube which is placed at the bifurcations, ensuring that the magnesium alloy tube always supports the lumens till the lumens are reconstructed, and improving the security of the stent made from magnesium alloy.

In a class of this embodiment, the barbs are disposed on the outer wall of the magnesium alloy tube. The barbs are staggered and opposite-distributed. The magnesium alloy tube is inserted in the lumen of the human body, and the barbs are inserted in a tube wall of the lumen, thus the magnesium alloy tube is connected to the lumen of the human body, and the lumen of the human body is stapled.

In a class of this embodiment, the barbs are conical, bottoms of the conical barbs are integrated with the magnesium alloy tube. The barbs are conical, thus it is convenient for the barbs to be inserted in the tube wall of the lumen of the human body. The barbs are opposite-distributed, thus the tube wall of the broken lumens is tensioned towards a center of the magnesium alloy tube.

In a class of this embodiment, the barbs are not limited to be conical. The barbs can be in other shapes. Optionally, the barbs comprise a cylindrical body and a conical top, and the cylindrical body is integrated with the magnesium alloy tube.

In a class of this embodiment, when the magnesium alloy tube is connected to the lumen of the human body, the magnesium alloy wire operates to fixedly connect the magnesium alloy tube to the lumen, thus preventing the magnesium alloy tube from falling off from the lumen or dislocating.

In a class of this embodiment, the magnesium alloy tube, the magnesium alloy wire, and the barbs are coated with a protection layer. The protection layer is a silver ion layer, or a biocompatible and anti-corrosion coating. The silver ion layer and the biocompatible and anti-corrosion coating feature strong bactericidal ability, prolongs the degradation time of the magnesium alloy, and ensures that the magnesium alloy tube always supports the lumens till the lumens are reconstructed, thus the security of the stent is improved

In a class of this embodiment, a main component of the magnesium alloy tube is magnesium, and the magnesium alloy tube contains iron, zinc, calcium, or a mixture thereof. Magnesium, iron, zinc, and calcium can be absorbed by the human body, thus bring no harm to the human body.

Advantages of the degradable stent according to embodiments of the invention are summarized as follows: An outer wall of the stent is provided with the barbs and the magnesium alloy wire. The magnesium alloy tube is inserted in the lumen of the human body, and the barbs are inserted in the inner wall of the lumen. The magnesium alloy wire is wound around the magnesium alloy tube and the lumen, thus the stent is fixedly connected to the lumen of the human body, preventing the magnesium alloy tube from falling off from the lumen of the human body, dislocating, or event collapsing as the magnesium alloy is absorbed by the human body. A joint between the main body and the branch is coated with a magnesium alloy protection layer, thus ensuring that the stent always supports the lumens till the lumens are reconstructed, and improving the security of the stent made from magnesium alloy.
FIG. **1** is a first schematic diagram of a degradable stent which is implanted in human body in accordance with one embodiment of the invention; and
FIG. **2** is a second schematic diagram of degradable stent which is implanted in human body in accordance with one embodiment of the invention.

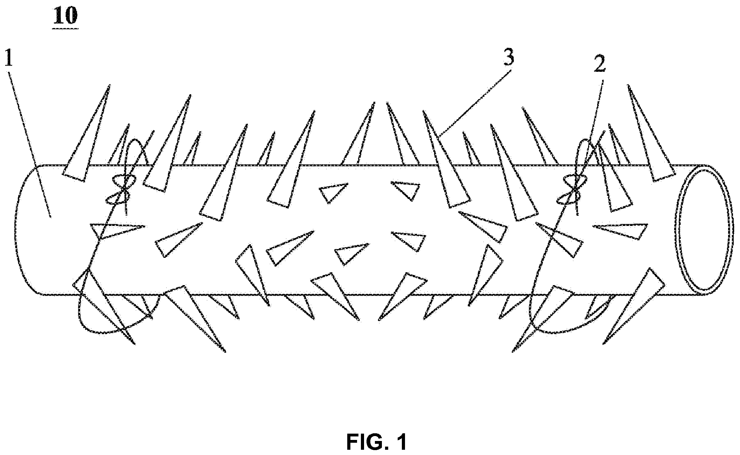

As shown in FIGS. 1-2, a degradable stent 10 which is implanted in human body comprises a cylindrical magnesium alloy tube 1 and a magnesium alloy wire 2. An outer wall of the magnesium alloy tube 1 is provided with a plurality of barbs 3. The magnesium alloy tube 1 is inserted in a lumen in human body, and the barbs 3 are inserted in an inner wall of the lumen in the human body. The magnesium alloy wire **2** is wound around the magnesium alloy tube **1** and the lumen, thus the stent **10** is fixedly connected to the lumen of the human body, preventing the stent **10** from falling off from the lumen of the human body, dislocating, or event collapsing as the magnesium alloy is absorbed by the human body.

The magnesium alloy tube **1,** the magnesium alloy wire **2,** and the barbs **3** are coated with a protection layer. The protection layer is a silver ion layer, or a biocompatible and anti-corrosion coating. The magnesium alloy contains iron, zinc, calcium, or a mixture thereof.

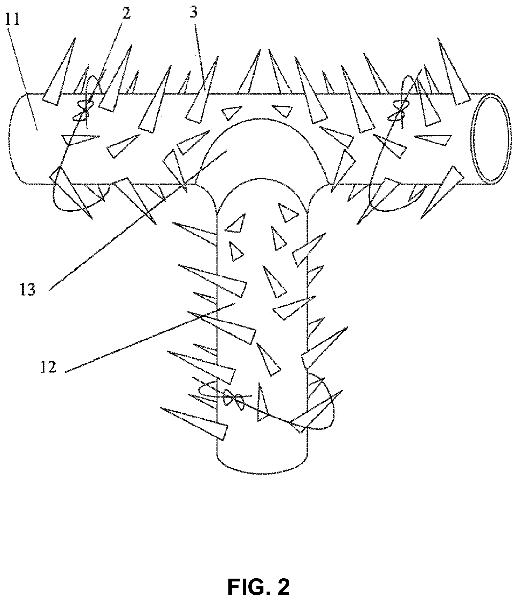

As shown in FIG. **2****,** the magnesium alloy tube comprises a main body **11** and at least one branch **12.** The main body **11** communicates with the branch **12.** Sizes and shapes of the main body **11** and the branch **12** are tailored according to actual conditions of the lumen in the human body, for example, the main body and the branch form a T-shaped bifurcation, a Y-shaped bifurcation, or an amorphous bifurcation. A joint between the main body **11** and the branch **12** is coated with a magnesium alloy protection layer **13.** Bifurcations of the lumens in the human body (including vessel, lymphatic tube, nerve, or biliary tract) are the positions which bear the most frequent blood rush or body fluid rush, thus the magnesium alloy tube placed at the bifurcations is more likely to break or collapse. Therefore, a magnesium alloy protection layer **13** is applied to the magnesium alloy tube which is placed at the bifurcations, ensuring that the magnesium alloy tube always supports the lumens till the lumens are reconstructed, and improving the security of the stent made from magnesium alloy. As shown in FIG. **1-2****,** the barbs **3** are disposed on the outer wall of the magnesium alloy tube. The barbs are staggered and opposite-distributed. The magnesium alloy tube **1** is inserted in the lumen of the human body, and the barbs **3** are inserted in a tube wall of the lumen, thus the stent **10** is connected to the lumen of the human body, and the lumen of the human body is stapled.

As shown in FIG. **1****,** the barbs **3** are conical, bottoms of the conical barbs are integrated with the magnesium alloy tube **1.** The barbs **3** are conical, thus it is convenient for the barbs to be inserted in the tube wall of the lumen of the human body. The barbs are opposite-distributed, thus the tube wall of the broken lumens is tensioned towards a center of the magnesium alloy tube.

The barbs **3** are not limited to be conical. The barbs can be in other shapes. Optionally, the barbs have a cylindrical body and a conical top, and the cylindrical body is integrated with the magnesium alloy tube.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A degradable stent, comprising:
a cylindrical magnesium alloy tube; and
a magnesium alloy wire;
wherein
in use, an outer wall of the magnesium alloy tube is provided with barbs; the magnesium alloy tube is inserted in a broken or damaged lumen of human body, and the barbs are inserted in an inner wall of the lumen; the magnesium alloy wire is wound around the magnesium alloy tube and the lumen, and the magnesium alloy tube is fixedly connected to the lumen of the human body.

2. The stent of claim 1, wherein the magnesium alloy tube comprises a main body and at least one branch; and the main body communicates with the branch.

3. The stent of claim 2, wherein a joint between the main body and the branch is coated with a magnesium alloy protection layer.

4. The stent of claim 2, wherein the main body and the branch form a T-shaped bifurcation, a Y-shaped bifurcation, or an amorphous bifurcation.

5. The stent of any one of claims 1-4, wherein the barbs are disposed on the outer wall of the magnesium alloy tube; and the barbs are staggered and opposite-distributed.

6. The stent of claim 5, wherein the barbs are conical, bottoms of the conical barbs are integrated with the magnesium alloy tube.

7. The stent of claim 5, wherein the barbs comprise a cylindrical body and a conical top, and the cylindrical body is integrated with the magnesium alloy tube.

8. The stent of claim 1, wherein the magnesium alloy tube, the magnesium alloy wire, and the barbs are coated with a protection layer; and the protection layer is a silver ion layer, or a biocompatible and anti-corrosion coating.

9. The stent of claim 1, wherein the stent contains magnesium, iron, zinc, calcium, or a mixture thereof.
